(19) Europäisches Patentamt
European Patent Office
Office européen des brevets

(11) **EP 1 664 747 B1**

(12) **EUROPÄISCHE PATENTSCHRIFT**

(45) Veröffentlichungstag und Bekanntmachung des Hinweises auf die Patenterteilung:
**25.04.2007 Patentblatt 2007/17**

(21) Anmeldenummer: **04766754.8**

(22) Anmeldetag: **09.09.2004**

(51) Int Cl.:
**G01N 21/82** *(2006.01)* **G01N 21/59** *(2006.01)*
**G01N 33/543** *(2006.01)*

(86) Internationale Anmeldenummer:
**PCT/EP2004/052109**

(87) Internationale Veröffentlichungsnummer:
**WO 2005/031325 (07.04.2005 Gazette 2005/14)**

(54) **VERFAHREN UND VORRICHTUNG ZUM NACHWEIS SEHR GERINGER PARTIKELMENGEN**

METHOD AND DEVICE FOR THE DETECTION OF VERY SMALL QUANTITIES OF PARTICLES

PROCEDE ET DISPOSITIF POUR IDENTIFIER DE TRES PETITES QUANTITES DE PARTICULES

(84) Benannte Vertragsstaaten:
**AT BE BG CH CY CZ DE DK EE ES FI FR GB GR HU IE IT LI LU MC NL PL PT RO SE SI SK TR**

(30) Priorität: **25.09.2003 DE 10344924**

(43) Veröffentlichungstag der Anmeldung:
**07.06.2006 Patentblatt 2006/23**

(73) Patentinhaber:
• **Odefey, Constantin**
**22303 Hamburg (DE)**
• **Werner, Friedrich**
**22397 Hamburg (DE)**

(72) Erfinder: **ODEFEY, Constantin**
**22303 Hamburg (DE)**

(74) Vertreter: **Becker Kurig Straus**
**Patentanwälte**
**Bavariastrasse 7**
**80336 München (DE)**

(56) Entgegenhaltungen:
**US-A- 5 100 805** **US-A- 5 534 441**

• **EIGEN M ET AL: "SORTING SINGLE MOLECULES: APPLICATION TO DIAGNOSTICS AND EVOLUTIONARY BIOTECHNOLOGY" PROCEEDINGS OF THE NATIONAL ACADEMY OF SCIENCES OF USA, NATIONAL ACADEMY OF SCIENCE. WASHINGTON, US, Bd. 91, Juni 1994 (1994-06), Seiten 5740-5747, XP002029412 ISSN: 0027-8424**

EP 1 664 747 B1

**Beschreibung**

Die vorliegende Erfindung stellt ein Verfahren und eine Vorrichtung zum Nachweis von Partikelmengen im femto- und attomolaren Bereich durch Erfassung von Antigen-Antikörper-Reaktionsprodukten bereit.

Verschiedene Verfahren zum Nachweisen geringer Partikelmengen sind im Stand der Technik bekannt, beispielsweise nephelometrische und turbimetrische Verfahren, ebenso wie als dynamic-light-scattering (DLS) bezeichnete Verfahren.

Bei nephelometrischen und turbidimetrischen Verfahren nutzt man den Tyndall-Effekt, bei dem die Beleuchtung eines kleinen Teilchens ein weitwinkliges Streulicht auslöst. Die Streuung des Lichtes kann entweder durch Messung der Intensitätsabnahme des einfallenden Lichtstrahles nach dem Durchgang durch das streuende Medium oder durch Bestimmung der Intensität des seitlich abgelenkten Lichtes ermittelt werden. Im ersten Fall spricht man von der Methode der Turbidimetrie oder Extinktionsmessung und im zweiten Fall von der eigentlichen Nephelometrie oder Tyndallometrie.

Einen anderen Vorgehensansatz bieten Verfahren, die als dynamic-light-scattering (DLS) Verfahren bezeichnet werden. Bei diesen Verfahren betrachtet man nur einen (oder wenige) Punkte auf der das Teilchen umspannenden Lichtkugel und wertet zusätzlich die durch die Brown'sche Molekularbewegung hervorgerufene Helligkeitsmodulation aus. Durch Fokussierung auf ein winziges Beobachtungsvolumen versucht man, die störenden Streulichtüberlagerungen mehrerer Teilchen zu reduzieren. Infolgedessen durchläuft ein Teilchen ein winziges beleuchtetes Volumen sehr schnell, so dass die auswertende Opto-Elektronik erhebliche Fluktuationsfrequenzen erkennen muss. Bei der aufwendigen Signalverarbeitung stehen viele Informationen zur Verfügung, so dass diese Systeme nur bedingt zur quantitativen Analyse einsetzbar sind.

Verfahren, bei denen Partikelaggregation über Antigen-Antikörperbindung zur Messung eines Analyten verwendet werden sind zum Beispiel aus US 5,534,441 und aus US 5,100,805 bekannt. Optische Methoden zur Detektion der dabei gebildeten Aggregate sind ebenfalls aus US 5,534,441 bekannt und beinhalten Lichtstreuung und auch Transmissionsmessungen.

Es sind weiterhin Verfahren bekannt, die die Größe eines Teilchens in Lösung über die Diffusionszeit in einem kleinen Lichtkegel bestimmen (EIGEN M ET AL: "SORTING SINGLE MOLECULES: APPLICATION TO DIAGNOSTICS AND EVOLUTIONARY BIOTECHNOLOGY", PROCEEDINGS OF THE NATIONAL ACADEMY OF SCIENCES OF USA, NATIONAL ACADEMAY OF SCIENCE, WASHINGTON, US, Bd. 91, Juni 1994 (1994-06), Seiten 5740-5747).

Die Nachweisverfahren des Stands der Technik, weisen darüber hinaus noch weitere Nachteile auf. Zunächst sei erwähnt, dass obwohl die Nachweisempfindlichkeit der vorstehend beschriebenen Verfahren in den letzten Jahren stark gestiegen ist, auf verschiedensten Gebieten weiterhin ein starker Bedarf nach Nachweisverfahren mit höherer Empfindlichkeit besteht. Darüber hinaus erfordern die Nachweisverfahren des Stands der Technik immer noch vergleichsweise hohe Probenmengen, was insbesondere bei medizinischtechnischen Verfahren mit Belastungen für die untersuchte Person verbunden sein kann.

Aufgabe der vorliegenden Erfindung ist daher die Bereitstellung eines Verfahrens zum Nachweis geringer Partikelmengen, das eine höhere Empfindlichkeit als die Verfahren des Stands der Technik aufweist. Darüber hinaus sollte ein derartiges Verfahren eine geringere Verdünnung der Probe und/oder eine geringere Probenmindestmenge erfordern, für eine Durchführung von Probenuntersuchungen im größeren Maßstab geeignet sein, und nach einer einfachen Schulung auch von Mitarbeitern ohne besondere Vorkenntnisse durchführbar sein. Des weiteren ist die Bereitstellung einer Vorrichtung zum Nachweisen von geringen Partikelmengen eine erfindungsgemäße Aufgabe.

Diese Aufgabe wird gelöst durch Bereitstellung eines Verfahrens zum Nachweisen von Partikelmengen im femto- und attomolaren Bereich durch Erfassung von Antigen-Antikörper-Präzipitaten, welches umfasst: Bereitstellen eines Probenfluids, das im wesentlichen Partikel mit einer bestimmten maximalen Partikelgröße enthält, wobei die Partikel mindestens zwei Antikörper-Bindestellen aufweisen; Bereitstellen eines Antikörper enthaltenden Fluids, das im wesentlichen Partikel einer bestimmten maximalen Partikelgröße enthält; Kontaktieren des Probenfluids mit dem Antikörper enthaltenden Fluid, wobei ein Reaktionsfluid erhalten wird, wobei der Antikörper in Gegenwart eines Partikels mit mindestens zwei Antikörper-Bindestellen ein Antigen-Antikörper-Präzipitat bilden kann; Führen eines Lichtstrahls durch das Reaktionsfluid; Erfassen eines Signals mittels einer Extinktionsmessung an der Hell-Dunkel-Grenze des Lichtkegels, der beim Durchgang des vom Laser erzeugten Lichts durch die das Reaktionsfluid enthaltende Messkammer entsteht, durch einen Photoempfänger, wobei die Signalstärke von der Größe und Zahl der gebildeten Antigen-Antikörper-Präzipitate abhängig ist und wobei Zahl und Größe der Partikel durch die Messung der Transitionszeit bestimmt wird.

Weiterhin stellt die vorliegende Erfindung einen Kit zum qualitativen und/oder quantitativen Nachweis eines bestimmten, nachzuweisenden Partikels bereit, wobei das bestimmte Partikel mindestens zwei Antikörper Bindestelle aufweist, wobei der Kit umfasst: mindestens einen Antikörper, der spezifisch an das bestimmte Partikel binden kann, und mindestens ein geeignetes Fluid zur Aufnahme der Probe, und eine Vorrichtung zur Erfassung geringer Partikelmengen, welche aufweist: einen Laser, eine Messkammer, und einen Photoempfänger, der zur Durchführung einer Extinktionsmessung an der Hell-Dunkel-Grenze des Lichtkegels ausgelegt ist, der bei Durchgang des vom Laser erzeugten Lichts durch die Partikel in einem Fluid enthaltende Messkammer entsteht, wobei der Photoempfänger bezüglich des Lasers derart angeordnet ist, dass der Laserstrahl knapp am Photoempfänger vorbei geht.

Kurze Beschreibung der Figuren

Fig. 1a-d verdeutlichen schematisch die Vorgänge bei der Bildung eines Antigen-Antikörper-Präzipitats unter Verwendung eines bivalenten Antikörpers.

Fig. 2a veranschaulicht ein Ergebnis einer Erfassung unter Verwendung des erfindungsgemäßen Verfahrens, wobei ein Partikel umfassendes Probenfluid nach Filtration mit einem Filter mit einer Porengröße von 200 nm (vor der Antikörper-Zugabe) untersucht wurde. Die Signale entsprechen Partikeln, deren Durchmesser kleiner als die verwendete Filtergröße ist. Die x-Achse zeigt die Teilchengröße und die y-Achse die Teilchenanzahl an.

Fig. 2b veranschaulicht ein Ergebnis einer Erfassung, wobei das Reaktionsgemisch untersucht wurde, das nach einem gleichzeitigem separatem Injizieren von mit einem Filter mit einer Porengröße von 200 nm gefilterten Lösungen des Probenfluids und des Antikörper-Fluids erhalten wird. In diesem Reaktionsgemisch erfolgt eine Reaktion und Mikropräzipitationen werden gebildet, die einen Durchmesser aufweisen, der größer als die verwendete Filterporengröße ist. Die x-Achse zeigt die Teilchengröße und die y-Achse die Teilchenanzahl an.

Fig. 3 zeigt eine Schemazeichnung einer Anlage zur Durchführung des erfindungsgemäßen Verfahrens.

Während der zahlreichen Versuche, die zu der vorliegenden Erfindung führten, gelang die Bereitstellung eines Verfahrens, bei dem die Empfindlichkeit um einen Faktor 1000 über der Empfindlichkeit vergleichbarer Verfahren des Stands der Technik liegt. Diese starke Steigerung der Empfindlichkeit beruht auf einem veränderten physikalischen Erfassungsverfahren, bei dem eine Signalerfassung mittels einer Extinktionsmessung an der Hell-Dunkel-Grenze des Lichtkegels, der beim Durchgang des vom Laser erzeugten Lichts durch die das Reaktionsfluid enthaltende Messkammer entsteht, durch einen Photoempfänger erfolgt, im Zusammenspiel mit einer daran angepassten analytischen Probenvorbereitung.

Darüber hinaus ermöglicht die vorliegende Erfindung eine Verringerung des Volumens der Messkammer um ungefähr einen Faktor 30 bis 50. Während bei Verfahren des Stands der Technik Messkammer-Volumen im Bereich von beispielsweise 1,6 ml benötigt werden, können bei dem erfindungsgemäßen Verfahren Messkammern mit einem Volumen im Mikroliterbereich (beispielsweise 40 μl) verwendet werden. Dies stellt einen erheblichen Vorteil bereit, da jede Probe zum Erhalt einer einheitlichen Matrix, welche beispielsweise gleiche Transparenz, Viskosität, etc. aufweist, verdünnt werden muss, so dass bei einem erfindungsgemäßen Verfahren im Vergleich zu Verfahren des Stands der Technik eine Probe weniger stark verdünnt werden muss.

Weiterhin gelingt es mit dem erfindungsgemäßen Verfahren die benötigte Mindestmenge an Probe zu verringern. Während Verfahren des Stands der Technik mehr als 100 μl benötigen, reichen bei den erfindungsgemäßen Verfahren um ein Vielfaches geringere Probenmengen (beispielsweise 3,5 μl) aus.

Der Begriff "Partikel" bezeichnet in der vorliegenden Anmeldung jedes dreidimensionale Gebilde, das einen anderen Brechungsindex als das Trägermedium hat.

Der Begriff "Präzipitation" beschreibt in der vorliegenden Anmeldung den Vorgang, dass bei einer Reaktion von löslichen Antigenen mit spezifischen Antikörpern, eine Bildung eines Antigen-Antikörper-Komplexes erfolgt, der eine geringere Löslichkeit in dem verwendeten Lösungsmittel als das verwendete Antigen beziehungsweise der verwendete Antikörper aufweist, was zunächst zu einer Trübung des Reaktionsgemisches und anschließend zu einer Sedimentation dieses Antigen-Antikörper-Komplexes führt.

Das erfindungsgemäße Verfahren ermöglicht einen Nachweis von geringen Partikelmengen. Beispielsweise bei niedermolekularen Stoffen, das heißt bei Stoffen mit einem Molekulargewicht von weniger als 500 g/mol, konnte bei Verwendung des erfindungsgemäßen Verfahrens eine Nachweisgrenze im Bereich von femto- und atto-Gramm pro Liter erreicht werden, während die Nachweisgrenze bislang üblicherweise im Mikro-, Nano-, oder Picogramm-Bereich pro Liter gelegen ist. Bei Stoffen mit einem Molekula gewicht im Bereich von über 500 g/mol liegt die Nachweisgrenze höher, beispielsweise bei Stoffen mit einem Molekulargewicht von 150 000 g/mol (z.B. IgG-Antikörper) liegt die Nachweisgrenze bei etwa 300 femtog/l. Dies bedeutet, dass das Probenfluid Partikel in der Größenordnung von femto- oder atto-Mol pro Liter enthalten kann.

In einem ersten Teilschritt des erfindungsgemäßen Verfahrens erfolgt die Bereitstellung eines Probenfluids, das im wesentlichen Partikel mit einer bestimmten maximalen Partikelgröße enthält. Dies kann beispielsweise auf zwei Wegen erreicht werden. Gemäß einer ersten Variante wird hierfür zunächst ein Fluid bereitgestellt, das im wesentlichen lediglich Partikel mit einer bestimmten maximalen Partikelgröße enthält und anschließend wird eine Probe, die im wesentlichen Partikel mit einer bestimmten maximalen Partikelgröße enthält, zu dem Fluid zugeführt. Gemäß einer zweiten Variante kann das Probenfluid gewonnen werden, in dem zunächst ein Fluid bereitgestellt wird, eine Probe zu dem Fluid zugeführt wird und anschließend Partikel, die eine bestimmte Partikelgröße überschreiten abgetrennt werden.

Die maximale Partikelgröße der Partikel in dem Probenfluid beziehungsweise in den anderen Fluida, die im wesentlichen lediglich Partikel einer bestimmten maximalen Partikelgröße enthalten, kann je nach gewünschter Anwendung gewählt werden. Bei einer Verwendung vieler geläufiger Antikörper kann die Abtrennung von Partikeln erfolgen, die größer als 20 - 450 nm, bevorzugter größer als 100 - 300 nm, insbesondere größer als 200 nm sind. Eine derartige Abtrennung kann beispielsweise durch Filter mit einer geeigneten, entsprechenden Porengröße von 20 - 450 nm, bevorzugter 100 - 300 nm, insbesondere 200 nm oder durch andere einem Fachmann bekannte Verfahren erfolgen. Wenn die Agglutination näherungsweise als Fläche betrachtet wird, so geht die Halbierung der Filtergröße in Bezug auf die Molekülanzahl, die nachweisbare Reaktionsprodukte liefert, quadratisch ein. Falls beispielsweise ein 100 nm Filter anstelle eines 200 nm Filters verwendet wird, so sind etwa nur ein Viertel der bei Verwendung eines 200 nm Filters benötigten Antigen-Antikörper-Moleküle notwendig, die miteinander reagieren müssen, um ein detektierbares Ergebnis zu erhalten. Darüber hinaus besteht beispielsweise bei Verwendung von 25 nm Filtern die Möglichkeit Antigen-Antikörper-Antigen Trimere nachzuweisen. Bei einer Verwendung von Filtern mit derart geringer Porengröße muss jedoch auf sorgfältiges Arbeiten geachtet werden, da bereits wenige Moleküle zu einer erfassbaren Reaktion führen.

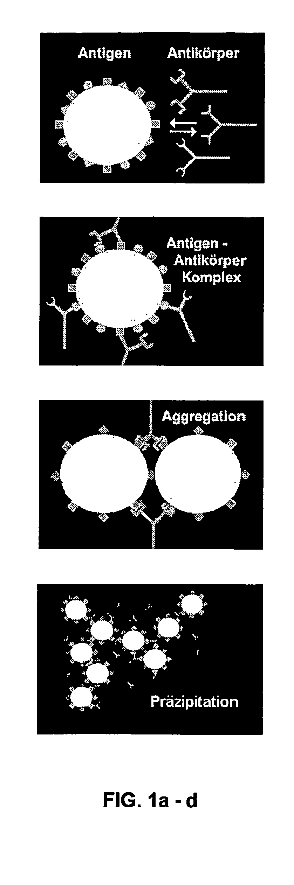

Damit eine Vernetzungsreaktion ablaufen kann, müssen die in dem Probenfluid enthaltenen Partikel über mindestens zwei Antikörper-Bindungsstellen verfügen und können somit als Antigen fungieren. Fig. 1a bis d veranschaulicht schematisch, dass körperfremde Stoffe oder Partikel, wie beispielsweise Bakterien, Viren, Toxine, Proteine, als Antigene fungieren und mit einem Antikörper nach dem "Schlüssel-Schloß-Prinzip" reagieren (Fig. 1b). Ein bivalenter Antikörper, wie beispielsweise ein IgG-Antikörper, bindet dabei zwei Antigene (Fig. 1c). Da jedes Antigen mehrere Antikörper binden kann, erfolgt hierbei eine Vernetzung (Präzipitation), die bei dem erfindungsgemäßen Verfahren erfasst wird (Fig. 1d). Für Antikörper mit höherer Valenz erfolgt eine Präzipitation in analoger Weise. Falls das verwendete Antigen eine höhere Anzahl an Antikörper-Bindungsstellen zur Verfügung stellt, so bietet dies den Vorteil, dass auf diese Weise der Ablauf einer Vernetzungsreaktion besser sicher gestellt werden kann.

Das erfindungsgemäße Verfahren eignet sich für beliebige Antigene, soweit sie über mindestens zwei Antikörper-Bindestellen aufweisen. Vorzugsweise sollten die untersuchten Partikel größer als etwa 10 Nanometer sein und sollten gleichzeitig kleiner als die gewählte Partikel-Maximalgröße sein. Moleküle, die kleiner als 10 nm sind können als Haptene wirken. Haptene sind inkomplette Antigene, d.h. ihre molekulare Größe reicht nicht aus, um eine Immunantwort auszulösen beziehungsweise eine Agglutination herbeizuführen. Diese niedermolekularen Stoffe sind zwar hochspezifisch in der Paratop-Bindungsstelle des Antikörpers, ragen aber nicht weit genug aus dieser Bindungsstelle heraus, dass ein zweiter Antikörper sich an ihnen binden könnte. Sie blockieren den jeweiligen Antikörper, ohne dass es zu einer Vernetzungsreaktion kommen kann. Größere Antigene, wie beispielsweise Bakterien, müssen vor der Messung chemisch oder physikalisch zertrümmert werden, was mit verschiedenen dem Fachmann bekannten Verfahren, beispielsweise durch eine Behandlung mit Ultraschall, Säuren, Laugen, Tensiden erfolgen kann. Dies stellt den Vorteil bereit, dass auf diese Weise aus nur einem einzigen Bakterium Dutzende von Bruchstücken gewonnen werden können, und es nicht mehr notwendig ist, einzelne Bakterien miteinander agglutinieren zu lassen. Die derart erhaltenen Bruchstücke, beispielsweise Oberflächenproteine, stellen wiederum kleinere Antigene dar und können mit dem passenden Antikörper eine spezifisch messbare Reaktion hervorrufen.

Darüber hinaus sollte das Antigen im verwendeten Puffer im wesentlichen löslich sein und sollte nur eine geringe Adsorptionsneigung an die Wände der verwendeten Vorrichtungen und Filter haben.

Das erfindungsgemäße Verfahren umfasst weiter die Bereitstellung eines Antikörper enthaltenden Fluids.

Bei dem erfindungsgemäßen Verfahren können prinzipiell alle gewünschten Antikörper zum Einsatz kommen. Antikörper, die sich für die Durchführung eines erfindungsgemäßen Verfahrens als besonders vorteilhaft erwiesen haben, sind beispielsweise das bivalente Immunglobulin G (IgG), oder das decavalente Immunglobulin M (IgM). Gemäß einem dem Fachmann bekannten Verfahren, können dabei Antikörper mit bestimmter Spezifität gewonnen werden. Darüber hinaus können auch je nach Typ des nachzuweisenden Partikels auch andere Antikörper zum Einsatz kommen, die zu einer anderen Klasse von Antikörpern zählen. Hierbei können die Antiköper monoklonal oder polyklonal sein. Bei Verwendung von monoklonalen Antikörpern können zwei auf verschiedene Antigene gerichtete, monoklonale Antikörper eingesetzt werden, um eine Präzipitation herbeizuführen. Wie vorstehend in Bezug auf das Antigen erwähnt, sollte auch der Antikörper im verwendeten Puffer im wesentlichen löslich sein und eine geringe Adsorptionsneigung an die Wände der verwendeten Vorrichtungen und Filter haben.

Bei dem erfindungsgemäßen Verfahren kann ein unerwünschter, zu starker Antigen- oder Antikörperüberschuss auf verschiedene Weisen, beispielsweise durch Durchführung geeigneter Verdünnungsreihen vermieden werden. Ein derartiger, zu starker Antigen- oder Antikörperüberschuss könnte sonst zu einer Präzipitationshemmung (zu einem sog. "Prozoneneffekt") führen, da bei einem starken Antikörperüberschuss jedes Epitop (Antigenbindungsstelle) nur einen einzigen Antikörper monovalent bindet und eine Kreuzvernetzung nicht mehr möglich ist, oder da bei einem zu starken Antigenüberschuss häufig das Trimere aus einem Antikörpermolekül und zwei Antigenmolekülen gebildet wird.

Als Fluid, sowohl für die Herstellung des Probenfluids, als auch für die Herstellung des Antikörper enthaltenden Fluids kann grundsätzlich jedes Gas oder jede Flüssigkeit verwendet werden. Bevorzugt ist das Fluid eine Flüssigkeit. Vielfach handelt es sich bei der Flüssigkeit um Wasser oder im Stand der Technik bekannte Pufferlösungen, wie beispielsweise PBS (phosphate buffered saline, phospatgepufferter Salzlösung), insbesondere wenn dem Analyseverfahren eine biochemische Reaktion zugrunde liegt. Grundsätzlich kann es sich bei der Flüssigkeit aber auch um eine andere transparente Flüssigkeit handeln, beispielsweise um flüssige Kohlenwasserstoffe, Säuren oder Laugen.

Bei dem erfindungsgemäßen Verfahren wird in einem nächsten Schritt das Probenfluid mit dem Antikörper enthaltenden Fluid in Kontakt gebracht, wobei der Antikörper in Gegenwart eines Antigens ein Antigen-Antikörper-Präzipitat bilden kann, das nun beispielsweise mit der erfindungsgemäßen Vorrichtung nachgewiesen werden kann.

Darüber hinaus ist es jedoch vielfach von Interesse, das Vorliegen von Haptenen in einer Probe zu ermitteln. Haptene sind nicht-komplette Antigene, d. h. sie sind zu klein, als dass mehr als ein Antikörper gebunden werden kann. Haptene können insbesondere Pharmaka, Drogen, Pestizide, Umweltgifte, Steroidhormone oder Mykotoxine sein. Haptene binden spezifisch an Antikörper. Sie blockieren jedoch lediglich die Paratop-Bindungsstelle des Antikörpers, ohne dass eine Kettenreaktion stattfinden kann. Die Mindestgrösse für Immunogene (komplette Antigene) liegt bei 5 bis 10 kDa, d. h. > 30 Aminosäurereste, d. h. > 3 nm Länge, denn ab dieser Grösse können mindestens zwei Antikörpermoleküle sich an diese Antigene mit entsprechend vorhandenen Epitop-Bindungsstellen koppeln und eine Kettenreaktion auslösen, die vielfach zu einer Präzipitation führt.

Eine Bestimmung von Haptenen kann beispielsweise mit hydrophilen makromolekularen Multispacern (hmM) oder mit verwandten, dem Fachmann bekannten Verbindungen erfolgen. Hydrophile makromolekulare Multispacer sind im Stand der Technik bekannt und umfassen ein hydrophiles Makromolekül, wie beispielsweise Albumin. An dieses hydrophile Makromolekül werden mit im Stand der Technik bekannten Spacern die gleichen Hapten-Moleküle oder verschiedene Hapten-Moleküle chemisch angekoppelt. Falls ein hydrophiler makromolekularer Multispacer mindestens zwei gleiche Hapten-Moleküle aufweist, so kann er für eine Präzipitation verwendet werden. Ein solches Molekül kann für einen Antikörper-Suchtest verwendet werden, der auf einer Verdrängungsreaktion basiert. Bei Haptenen liegt somit ein auf einer Verdrängungsreaktion basierendes Messprinzip vor, wie nachstehend beispielhaft beschrieben, wobei nur bei negativ-Proben Reaktionspeaks gesehen werden, d.h. nur bei negativ-Proben ein Nachweis von Reaktionsprodukten erfolgt.

Wenn eine Blut- oder Speichelprobe auf ein Hapten, beispielsweise Kokain untersuchen werden soll, so gibt man zu dem verdünnten Blut- oder Speicheltröpfchen einen Antikörper, der gegen Kokain gerichtet ist. Nach etwa einer Minute wird eine kleine Menge eines synthetisch hergestellten Kokain-hmM, d.h. ein hydrophiles, über Spacer mit Kokainmolekülen verknüpftes Makromolekül, hinzugegeben.

Wenn Kokain in der Probe vorliegt, wirkt dieses als Hapten und blockiert die Antikörperbindungsstellen. Die nachfolgende Zugabe des Kokain-hmM's zeigt keine Wirkung. Wenn jedoch kein Kokain in der Probe war, so wurden keine Antikörperbindungsstellen blockiert und die Zugabe von Kokain-hmM führt zu einer Kettenbildung, die als Teilchenwachstum mit dem erfindungsgemäßen Verfahren, beispielsweise mit der nachstehend beschriebenen Q-MAP-Vorrichtung, gemessen werden kann.

Bei dem erfindungsgemäßen Verfahren wird ein Lichtstrahl, insbesondere kohärentes Licht, wie beispielsweise ein Laserstrahl durch die zu untersuchende Flüssigkeit gestrahlt. Nachstehend wird das erfindungsgemäße Verfahren unter Bezugnahme auf einen Laserlichtstrahl beschrieben, dies schließt jedoch nicht aus, dass auch andere dem Fachmann bekannte, geeignete Lichtquellen bei dem erfindungsgemäßen Verfahren eingesetzt werden können. Beispielsweise kann ein Laserstrahl von der nachstehend detailliert beschriebenen, erfindungsgemäßen Vorrichtung, die auch als Q-MAP (quantitative measurement of attomolar precipitationproducts) - Vorrichtung bezeichnet wird, bereitgestellt werden. Alternativ können jedoch auch andere Vorrichtungen auf Basis der Offenbarung der hier vorliegenden Erfindung verwendet werden, die von einem Fachmann ausgehend von der Lehre der vorliegenden Erfindung entwickelt werden können.

Der Laser sendet einen Strahl durch die zu untersuchende Flüssigkeit und ermittelt die Anzahl der darin vorhandenen Teilchen und deren Größe. Hierbei erfolgt eine Extinktionsmessung an der Hell-Dunkel-Grenze des Lichtkegels, der beim Durchgang des vom Laser erzeugten Lichts durch die das Reaktionsfluid enthaltende Messkammer entsteht, durch einen Photoempfänger (wobei der Photoempfänger eine einstellbare Verstärkung des Signals, sowie einen einstellbaren Arbeitspunkt aufweisen kann), so dass die Vorrichtung als eine "molekulare Lichtschranke" wirkt. Eine Q-MAP-Vorrichtung wie nachstehend beschrieben kann beispielsweise Teilchengrößen zwischen 20 nm und 5 $\mu$m bestimmen, wobei jedoch aus dieser Messung keinerlei Aussagen über die Beschaffenheit beziehungsweise die Zusammensetzung der gemessenen Teilchen gemacht werden können. Laser und Photoempfänger sind nahezu in einer Achse angeordnet. Der Laserstrahl geht ganz knapp am Photoempfänger vorbei. Das vorwärts gestreute Licht bildet einen Kegel, auf dessen Hell-Dunkel-Grenze der Photoempfänger eingestellt wird.

Jedes Teilchen, das sich in einem Fluid in der Messkammer befindet und den Laserstrahl passiert, erzeugt ein Signal, wobei die Anzahl der Signale der statistischen Verteilung der Partikel in der Messkammer entspricht. Wenn Teilchen, auch kleinste Teilchen, im Focus des Lasers durch den Lichtkegel wandern, dunkeln sie ihn ab, quasi als Schattenwurf. Die Änderung der Ursprungshelligkeit (ohne Teilchenschatten) wird gemessen. Ein schneller Rechner, beispielsweise ein Pentium-Rechner, kann bis zu 10.000 Teilchendurchgänge pro Sekunde unterscheiden. Kleine Teilchen bewegen sich schneller als Große, die Transitionszeit ist ein direktes Maß für die Teilchengröße.

Falls mehrere Teilchen gleichzeitig im Laserstrahl sind, wird nur das Größte gemessen. Anhand der unterschiedlichen Geschwindigkeiten mit denen die Partikel den Laserstrahl passieren, kann man die jeweilige Teilchengröße über die dem Fachmann wohlbekannte Stokes-Einstein-Gleichung ermitteln. Bei dieser Messung ist weniger die absolute Teilchengröße, als die Änderung der Teilchengröße (die durch das Wachstum von Teilchen hervorgerufen wird) von Wichtigkeit. Der jeweilige Filter dient als Maß zur Bestimmung des Beginn von Teilchenwachstum. Je größer die Filterporen sind, desto größer müssen die Präzipitate werden, um sich vom "Grundrauschen" zu unterscheiden. Bei 100-200 nm Filtern reichen sehr wenige wachsende Teilchen, um ein messbares Signal zu erzeugen. Da die Teilchen statistisch verteilt sind, findet auch immer im Laserfokus, der in der Mitte der Messkammer ist, ein Teilchenwachstum statt.

Die Geschwindigkeit mit der die Teilchen den Laserstrahl passieren wird erfasst, in dem die Transitionszeit gemessen wird, in der das Teilchen den Strahl durchläuft (vom Beginn der Helligkeitsänderung bis zum Ende derselben).

Hierbei zeigt sich, dass je sauberer die Lösung ist, desto wahrscheinlicher Teilchen einzeln im Laserstrahl vorliegen beziehungsweise, dass je verschmutzter die Lösung ist, sich desto mehr die Signale überlagern, was zu einer verminderten Empfindlichkeit führt.

Die Signalstärke ist abhängig von der Größe und Anzahl der Antigen-Antikörper-Präzipitate.

Bei konstanter Antikörperkonzentration steht die Abnahme des Meßsignals im direkten Zusammenhang mit der Antigenkonzentration.

Bei einer erfindungsgemäßen Erfassung kann dabei beispielsweise wie folgt vorgegangen werden: Alle zu untersuchenden Fluids werden durch Filter mit bestimmter Porengröße in die Messkammer injiziert, so dass bei einer Erfassung des Probenfluids oder des Antikörper-Fluids, jeweils allein, nur Signale bei einer Auswertung auftreten, die Partikel zeigen, die kleiner als eine bestimmte Teilchengröße sind. Bei dem erfindungsgemäßen Verfahren weisen sowohl die verwendeten Antikörper, als auch die verwendeten Antigene, eine Größe unterhalb der bestimmten Partikelgrößen-Grenze auf und sind somit kleiner als die Porengröße des zur Abtrennung verwendeten Filters (beispielsweise kleiner als 200 nm), so dass sie bei der Abtrennung nicht entfernt werden.

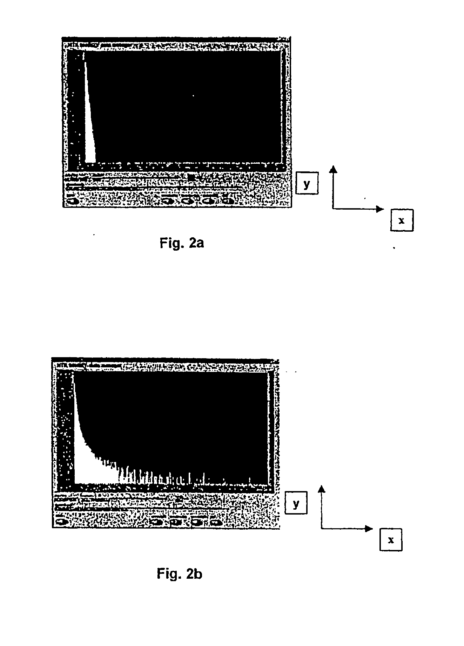

Ein Beispiel für ein Bild, das bei einer derartigen Erfassung eines Probenfluids auf Basis einer NaCl-Lösung erhalten wird, zeigt Fig. 2a. Bei diesen Bildern ist entlang der x-Achse die Teilchengröße und entlang der y-Achse die Teilchenanzahl veranschaulicht. Wie in Fig. 2a ersichtlich, sind nur Partikel bis zu einer bestimmten Maximalpartikelgröße vorhanden, wobei die Teilchenanzahl abnimmt, je größer die Partikelgröße ist. Ein analoges Erfassungsergebnis wird für das filtrierte und somit im wesentlichen nur Partikel einer bestimmten Partikelgröße enthaltende Antikörper-Fluid erhalten werden (nicht gezeigt).

Nach einem gleichzeitigen, separaten Injizieren des Probenfluids beziehungsweise des Antikörper-Fluids erfolgt eine Reaktion in der Messkammer unter Bildung von Mikropräzipitationen und eine Erfassung der Größe und Zahl der gebildeten Antigen-Antikörper-Präzipitate.

Ein derartiges Messergebnis ist beispielsweise in Fig. 2b veranschaulicht, worin das Auftreten von Teilchen erkennbar ist, die größer als die bestimmte Partikelgrößengrenze von beispielsweise 200 nm sind. Die Bildung von größeren Teilchen zeigt also an, dass eine Reaktion stattgefunden hat. Falls dagegen keine Reaktion zu größeren Teilchen stattfindet, ist die untersuchte Flüssigkeit frei von dem jeweiligen Antigen.

Die Messungen können zu jedem Zeitpunkt nach dem Befüllen der Reaktionskammer mit Probenfluid und Antikörper-Fluid begonnen werden. Gemäß einer Ausführungsform der vorliegende Erfindung werden die Messungen erst beispielsweise 60 Sekunden nach dem Befüllen der Messkammer gestartet, da leichte Schwankungen bei der Injektionsgeschwindigkeit zu Konvektionsunterschieden in der Messkammer führen können. Nach beispielsweise weiteren 60 Sekunden ist das Präzipitationsmaximum erreicht. Die danach eintretende Abnahme des Messsignals ermöglicht eine genäherte quantitative Erfassung oder Abschätzung der unterschiedlichen Antigen-Konzentrationen. Hierbei spielt die Ausgangskonzentration beider Reaktionsteilnehmer eine entscheidende Rolle. Je höher diese Ausgangskonzentrationen sind, desto länger wird die Zeit bis zur messbaren Abnahme des Präzipitationsmaximums.

Bei der Durchführung des erfindungsgemäßen Verfahrens ist es vorteilhaft, wenn die verwendeten Fluids eine möglichst hohe Reinheit aufweisen und beispielsweise mit einem Filter geeigneter Porengröße, beispielsweise von 200 nm, vor ihrer Verwendung filtriert wurden, um ein Grundrauschen bei der Erfassung zu eliminieren beziehungsweise zu minimieren. Darüber hinaus sollten die Materialien der während des erfindungsgemäßen Verfahrens verwendeten Vorrichtungen möglichst wenig Partikel abgeben. Beispielsweise kann die Messkammer aus PTFE (Polytetrafluorethylen) ausgebildet sein. Um zu verhindern, dass die verwendeten Materialien (beispielsweise der Messkammer) auf Dauer Teilchen abgeben, kann weiterhin die Messzeit so kurz wie möglich gehalten werden.

Darüber hinaus sollte die Konzentrationen der verwendeten Antigen- oder Antikörper-Lösungen so gering sein, dass bei einer stattfindenden Antigen-Antikörper-Reaktion nicht zu viele oder zu große Präzipitate entstehen, da sich sonst mehrere Teilchen auf einmal im Strahlengang befinden können und eine Transitionszeit nur sehr schwer ermittelbar ist. Die höchste Empfindlichkeit des erfindungsgemäßen Verfahrens ist im femto- und attomolaren Bereich.

Von besonderem Vorteil ist, dass das erfindungsgemäße Verfahren auch quantitative beziehungsweise halb-quantitative Aussagen ermöglicht.

Zwei Verfahren können beispielsweise zur Auswertung einer quantitativen Erfassung verwendet werden. Bei dem ersten Auswertungsverfahren wird die Fläche (F) unter der Messkurve zum Zeitpunkt t1 und t2 (beispielsweise zum Zeitpunkt t1=120 Sekunden und t2=180 Sekunden) ermittelt, wobei man über

$$F_N = (F_{t1} + F_{t2}):2,$$

einen guten Näherungswert $F_N$ für eine Fläche erhält, aus der dann ein quantitativer Wert für die Partikelanzahl erhalten werden kann. Bei diesen extrem niedrigen Konzentrationen ist das Teilchenwachstum linear, d.h. beispielsweise eine Verdopplung der Konzentration fährt zu einer doppelt so großen Fläche eines Messsignals.

Eine alternative und/oder ergänzende Methode zum Erhalten einer quantitativen Aussage, beruht darauf, dass die Lösungen so lange verdünnt werden, bis man an die kinetische Grenze der bimolekularen Reaktion stößt. Bei Konzentrationen unter 100 attomolar werden die freien Weglängen der Reaktionsteilnehmer zu groß (>100 $\mu$m) und es kommt in der vorgegebenen Messzeit zu keiner messbaren Präzipitation. Bei Konzentrationen über 10 nanomolar beginnt die sterische Behinderung durch zu zahlreiche und zu große Reaktionsprodukte und außerdem setzt der vorstehend erläuterte "Prozoneneffekt" ein. Bei einer z.B. 10 mikromolaren Lösung beträgt die freie Weglänge eines Teilchens nur noch 100 nm.

Der ermittelte Verdünnungsgrad an diesem physikalischen Endpunkt ist nur noch abhängig von Temperatur und Konvektion, da die Viskosität bei den hohen Verdünnungen z.B. in PBS (phosphate buffered saline, phospatgepufferter Salzlösung) keine Rolle spielt. Wenn diese beiden Parameter konstant gehalten werden, ist die Stärke der Verdünnung der Ausgangslösungen ein direktes Maß für die Konzentration der jeweiligen Lösungen.

Eine exakte quantitative Konzentrationsbestimmung von Proben ist mit dem erfindungsgemäßen Verfahren mittels einer Eichlösung mit bekannter Konzentration des jeweiligen Proteins möglich, wobei einem Fachmann bekannte Vorgehensweisen angewendet werden.

Nach einer weiteren Ausgestaltung des Verfahrens können kontinuierliche und/oder mehrfach stichprobenartige Messungen des Fluids in der Messkammer vorgenommen werden. Hierdurch ist es insbesondere möglich, den Endpunkt der Reaktion festzustellen und Mittelwerte der Erfassungen zu bilden. Bevorzugt wird bereits das von Teilchen mit einer Partikelgröße über dem bestimmten Wert im wesentlichen freie Trägerfluid vor Zugabe des Antikörpers und/oder vor Zugabe der Probe einer Messung unterzogen und das Ergebnis dieser Messung als Bezugswert für die Messung des Präzipitats herangezogen.

Bei dem erfindungsgemäßen Verfahren kommt man mit verhältnismäßig geringen Mengen Fluid aus und der Aufwand für die Entfernung von Stoffen, deren Partikelgröße einen bestimmten Wert überschreitet, aus dem verwendeten Fluid kann gemindert werden. Für die Entfernung solcher Stoffe kann das verwendete Fluid beispielsweise auch durch Umschalten von Ventileinrichtungen durch einen Bypass mit einem Filter geführt werden, der die besagten Stoffe ausfiltert. Das Filtern des verwendeten Fluids kann vor dem Einspeisen der Probe über eine bestimmte, vorgegebene Zeit durchgeführt werden, um sicherzustellen, dass keine die Messung störenden Stoffe mehr enthalten sind. Aus der Probe können Stoffe, deren Partikelgröße den bestimmten Wert überschreitet, insbesondere durch Filter entfernt werden, die in die Einspeisestellen integriert beziehungsweise diesen vorgeordnet sind. Auch nach dem Einspeisen der Probe kann das derart erhaltenen Probenfluid über eine bestimmte Zeit gefiltert werden, um störende Stoffe, die mit der Probe oder aus dem Leitungssystem in das Fluid gelangen könnten, zu entfernen.

Gemäss einer weiteren Ausgestaltung des Verfahrens wird das Trägerfluid nach Zugabe eines Antikörpers, die zu einem Nachweis oder nicht zu einem Nachweis eines Antigens geführt hat, erneut gefiltert, bis ein anderer Antikörper zugegeben wird. Hierdurch können zwischenzeitlich in das Trägerfluid eingetretene Stoffe, welche die Messung stören könnten, entfernt werden. Außerdem ist es hierdurch möglich, ein nachgewiesenes Reaktionsprodukt aus dem verwendeten Fluid zu entfernen, um eventuelle weitere Komponenten der Probe zu analysieren.

Das erfindungsgemäße Verfahren ist für vielfältige Anwendungen im Bereich der Wasseranalytik (Nachweis von Inhalts- und Schadstoffen), der Lebensmitteltechnologie (Nachweis von Mikroorganismen, Inhaltsstoffen) oder bei biologischen oder medizinischen Tests (z. B. Nachweis bestimmter DNA- oder RNA-Sequenzen, Bakterien oder von Allergenen (Allergietests)) geeignet. Weiterhin sind auch Anwendungen im Bereich hydrophiler makromolekularer Multi-Spacer, Branch-DNA-Sonden oder einer quantitativen PCR möglich. Insbesondere kann das erfindungsgemäße Verfahren auch zum Nachweis des infektiösen Prionen-Proteins PrP$^{Sc}$ bei BSE-Tests verwendet werden. Da bei dem erfindungsgemäßen Verfahren äußerst geringe Mengen dieses Prionen-Proteins nachgewiesen können, eignet sich das erfindungsgemäße Verfahren auch für einen Nachweis des infektiösen Prionen-Proteins PrP$^{Sc}$ aus Blut.

Darüber hinaus bietet das erfindungsgemäße Q-MAP-Messverfahren eine schnelle und einfache Möglichkeit, das Adsorptionsverhalten von verschiedenen Proteinen an unterschiedlichen Oberflächen zu testen. Hochpolierte Flächen könnten so zerstörungsfrei auf einen "Proteinadsorptionswert" überprüft werden, wobei Abweichungen vom Sollwert auf einen Oberflächendefekt hindeuten. So können beispielsweise bei aufgedampften, dünnen Metalloberflächen auf diese Weise Oberflächendefekte einfach und schnell nachgewiesen werden.

Weiterhin kann die vollständige Immobilisierung bestimmter Proteine an der jeweiligen Oberfläche ebenfalls geprüft werden, was beispielsweise im Bereich der DNA-Analytik und der Biochips-Technologie von hoher Bedeutung ist.

Eine weitere mögliche Anwendungsmöglichkeit des erfindungsgemäßen Verfahrens kann darin beruhen, dass der Einfluss von Chemikalien auf eine Oberfläche bestimmt werden kann. Insbesondere kommen beispielsweise Vorher-Nachher-Tests zum Nachweis von Veränderungen durch eine Oberflächenbehandlung in Betracht, da eine verätzte Fläche eine größere Oberfläche als eine nicht verätzte aufweist und somit eine größere Menge an Antigen-Material, beispielsweise Proteinen, an diese Oberfläche adsorbierbar ist. Hierbei kann beispielsweise die Menge der frei in Lösung gebliebenen Proteine bestimmt werden.

Noch eine andere Anwendungsmöglichkeit beruht darin, dass das erfindungsgemäße Verfahren ermöglicht, dass die Beschaffenheit auch der inneren Oberfläche von Schläuchen und Rohren getestet werden kann.

Nach Beendigung des erfindungsgemäßen Verfahrens können die Antigen-Antikörper-Präzipitationen aufgelöst werden, beispielsweise mit Proteinase K bei Substanzen die von Proteinase K nicht zerstört werden (z.B. Prionen und Substanzen, die keine Aminosäuren enthalten), und weiteren Behandlungs- oder Untersuchungsschritten unterworfen werden.

Ein weiterer wichtiger Vorteil des erfindungsgemäßen Verfahrens ist, wie vorstehend ausführlich beschrieben, dass es auch quantitative Erfassungen ermöglicht. Darüber hinaus kann das Verfahren vollautomatisch ausgeführt werden, was zu einer deutlichen Einsparung an Arbeitskosten führt, so dass somit ein vergleichsweise kostengünstig ausführbares Verfahren bereitgestellt wird.

Weiterhin ermöglicht das erfindungsgemäße Verfahren einen Nachweis von Krankheitserregern aus Körperflüssigkeiten oder -ausscheidungen, insbesondere Blut, in sehr geringen Mengen. Dies stellt einen sehr wichtigen Vorteil dar, da geringe Mengen an Blut, beispielsweise ein kleines Bluttröpfchen (ca. 10-20 µl), einfach an einer Fingerkuppe oder einem Ohrläppchen mit einem Röhrchen entnommen werden kann, ohne dass hierzu ein Arzt oder eine ausgebildete Krankenschwester erforderlich wäre. Derartige Tests können beispielsweise einfach in einer Apotheke oder aber auch in Altersheimen oder Rehabilitationseinrichtungen vorgenommen werden und liefern einfach und schnell eine Aussage darüber ob eine Infektion mit einem bestimmten Bakterium vorliegt. Von besonderem Vorteil ist, dass sowohl die Probenentnahme, als auch die Durchführung des erfindungsgemäßen Verfahrens mit der erfindungsgemäßen Vorrichtung keine medizinisch ausgebildeten oder hoch qualifizierten Mitarbeiter erfordert. Alternativ können mit einem derartigen Test natürlich auch andere Substanzen im Blut mit spezifischen Antikörpern ermittelt werden, beispielsweise Drogen oder Arzneimittel.

Während bei handelsüblichen Messgeräten das Verhältnis zwischen Antigen und Antikörper nicht mehr als um den Faktor 2-3 differieren darf, sind mit Q-MAP auch dann noch Messungen möglich, wenn von dem Idealmischungsverhältnis zwischen Antigen und Antikörper um den Faktor 100 abgewichen wird. (Bei gleich bleibender Antikörperkonzentration wurde die ideale Antigenkonzentration auf 1 % gesenkt und bis auf 10000% erhöht. Das gleiche wurde umgekehrt auch bei gleich bleibender Antigenkonzentration durchgeführt). Diese interessante Abweichung von der "Heidelberger-Kurve" im femto- und attomolaren Bereich, die durch die geringeren sterischen Behinderungen der Moleküle verursacht wird, macht zeitaufwändige Verdünnungsreihen überflüssig.

Insbesondere eignet sich das erfindungsgemäße Verfahren für eine Untersuchung von gepoolten Proben, was insbesondere für Untersuchungen von Blutkonserven (beispielsweise hinsichtlich HIV oder Hepatitis) und BSE-Testproben von Interesse ist. Mit dem erfindungsgemäßen Verfahren können etwa 50 Messungen pro Stunde durchgeführt werden, was bedeutet, dass bei einem 8-10 Stunden Tag mindestens 400 Untersuchungen pro Tag beziehungsweise mehr als 80.000 Messungen pro Jahr durchgeführt werden können. Bei 10-100 gepoolten Proben je Messung kann man also 800.000 - 8.000.000 Proben mit nur einem Messgerät pro Jahr untersuchen.

Bei einer Untersuchung von Blutkonserven, können beispielsweise 10 verschiedene Antikörper (gegen 10 verschiedene Krankheiten) auf einmal zu einer Blutkonserve zugesetzt werden, um festzustellen, ob diese Blutkonserve verwendbar ist Bei einer positiven Reaktion muss die Blutkonserve weggeworfen werden, da es egal ist, ob sie beispielsweise mit HIV oder Hepatitis verseucht ist. Falls es darüber hinaus von Interesse ist, welcher Erreger die positiv-Reaktion ausgelöst hat, so können die Antikörper einzeln zugegeben werden. Wenn man beispielsweise 100 Blutkonserven poolt und gleichzeitig 10 verschiedene Antikörper verwendet, so können mit nur einer Messung, die etwa 60 Sekunden dauert, festgestellt werden, ob alle 100 Blutkonserven verwendbar sind.

Ein Test, bei dem eine derart geringe Produktmenge ausreicht, und der eine entsprechend hohe Empfindlichkeit bis in den femto- oder attomolaren Bereich hin aufweist, ist bislang im Stand der Technik nicht bekannt und stellt einen wichtigen Fortschritt dar.

Darüber hinaus umfasst die vorliegende Erfindung ein Computerprogramm-Produkt, welches Programmcode-Mittel umfasst, die auf einem Computer-lesbaren Medium gespeichert sind, um das erfindungsgemäße Verfahren ausführen zu können, wenn das Computerprogramm-Produkt auf einem Computer, einer Netzwerk-Vorrichtung oder einer Vorrichtung, insbesondere einer analytischen Erfassungsvorrichtung betrieben wird. Weiterhin stellt die vorliegende Erfindung auch ein Computerprogramm-Produkt bereit, welches einen Programmcode umfasst und von einem Server herunterladbar ist, um das erfindungsgemäße Verfahren ausführen zu können, wenn das Computerprogramm-Produkt auf einem Computer, einer Netzwerk-Vorrichtung oder einer Vorrichtung, insbesondere einer analytischen Erfassungsvorrichtung (beispielsweise einer in der vorliegenden Anmeldung beschrieben Erfassungsvorrichtung) betrieben wird.

Die vorliegende Erfindung betrifft weiter eine Vorrichtung zum Nachweisen von geringen Partikelmengen, die auch als Q-MAP (quantitative measurement of attomolar precipitation-products) - Vorrichtung bezeichnet wird.

Eine derartige Vorrichtung umfasst eine Lichtquelle, wobei als Lichtquelle bevorzugt ein Laser verwendet wird.

Die Messkammer einer derartigen Vorrichtung sollte aus einem Material hergestellt sein, das im wesentlichen keine Partikel abgibt und das einen Durchgang eines Lichtstrahls, insbesondere eines Laserstrahls, ermöglicht. Derartige Materialien sind einem Fachmann bekannt. Beispielsweise kann die Messkammer aus PTFE (Polytetrafluorethylen) ausgebildet sein. Die Messkammer weist ein Volumen von weniger als 100 $\mu$l, vorzugsweise von 30 - 50 $\mu$l, insbesondere von 40 $\mu$l auf.

Die erfindungsgemäße Vorrichtung weist insbesondere einen Photoempfänger auf, der eine einstellbare Signalverstärkung und einen einstellbaren Arbeitspunkt aufweist.

Der Photoempfänger kann beispielsweise ausgewählt werden aus der Gruppe bestehend aus thermischen Detektoren, Photodioden, insbesondere Photoleiterdetektoren, Photovoltaischen Detektoren, Lawinendioden, Diodenarrays, Photomultipliem.

Eine erfindungsgemäße Erfassungsvorrichtung ermöglicht insbesondere eine Bestimmung von Teilchen zwischen 20 nm und 5 $\mu$m.

Zusätzlich stellt die vorliegende Erfindung auch einen Kit zum qualitativen und/oder quantitativen Nachweis eines bestimmten, nachzuweisenden Partikels bereit, beispielsweise eines Proteins oder Hormons, wobei die bestimmten Partikel mindestens zwei Antikörper-Bindestellen aufweisen. Der Kit umfasst eine Erfassungsvorrichtung, wie vorstehend beschrieben, mindestens einen Antikörper, der spezifisch an das bestimmte Partikel binden kann, und mindestens ein geeignetes Fluid zur Aufnahme der Probe. Ein derartiger Kit kann speziell auf die bestimmten Bedürfnisse eines Anwenders ausgelegt werden, und enthält aufeinander abgestimmte Komponenten und eine entsprechende Beschreibung für den Benutzer, der mit diesem Kit erfindungsgemäße Erfassungen sofort und in sehr einfacher Weise durchführen kann. Beispielsweise kann ein derartiger Kit auf die Bestimmung eines bestimmten Krankheitserregers in einer geringen Menge Blut oder auf die vorstehend beschriebenen Anwendungen zur Oberflächenuntersuchung verwendet werden.

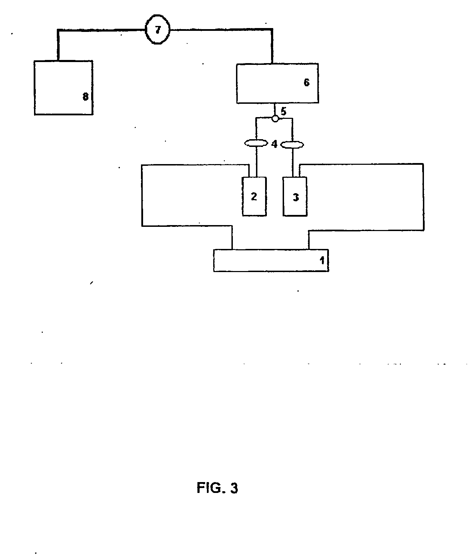

## Ausführliche Beschreibung von Figur 3

Die Erfindung wird nachfolgend anhand der anliegenden, beispielhaften, nicht-einschränkenden Schemazeichnung einer Anlage zum Durchführen des erfindungsgemäßen Verfahrens näher erläutert.

Der Probenwechsler 1 kann sowohl verschiedene Antigenlösungen (z.B. Blutproben), als auch unterschiedliche Antikörperlösungen in die jeweiligen Mischgefäße 2 beziehungsweise 3 injizieren, um eine Probe nacheinander auf verschiedene mögliche Erreger zu untersuchen. Die Mischgefäße 2 beziehungsweise 3 für die Antigen- beziehungsweise Antikörperlösungen können nicht nur zur Verdünnung der jeweiligen Lösungen dienen, sondern auch zum Spülen der Messkammer mit Puffer (PBS) oder Antikörperlösung.

Die Filter 4 sind austauschbar und weisen beispielsweise eine Porengröße von 200 nm auf. Das Ventil 5 kann so geschaltet werden, dass die Lösungen einzeln oder gemeinsam in die Messkammer 6 fließen können, worin permanent, einfach oder mehrfach stichprobenartig eine Erfassung mit der Erfassungsvorrichtung erfolgt.

Die Pumpe 7 ist z.B. eine kleine Vakuumpumpe die alle Lösungen ansaugt. Sie ist mit dem Ventil 5 koordiniert und pumpt nach erfolgter Messung den Inhalt der Messkammer in das Abfallgefäß 8. Dieses kann z.B. mit einer desinfizierenden bzw. keimtötenden Flüssigkeit versehen sein, um alle möglicherweise gefährlichen Stoffe sofort unschädlich zu machen.

Falls der eingespeiste Antikörper mit einem Antigen aus der Probe zum einem Antigen-Antikörper-Präzipitat reagiert, bilden sich Teilchen mit einer Partikelgröße, die den bestimmten Wert überschreitet. Diese Präzipitate erzeugen ein Signal, das sich deutlich von den Signalen eventuell noch im Trägerfluid vorhandener Partikel mit einer Partikelgröße unterhalb des bestimmten Werts unterscheidet. Infolgedessen werden diese Reaktionsprodukte eindeutig durch die Erfassungsvorrichtung nachgewiesen. Aus dem Nachweis ergibt sich, dass ein bestimmter Stoff in der Probe enthalten ist und gegebenenfalls kann, gemäß den vorstehend beschriebenen Methoden, auch dessen Gehalt ermittelt werden.

Falls nach Injektion einer ersten Antikörperlösung die Erfassungsvorrichtung keine Partikel oberhalb der bestimmten Partikelgröße nachweist oder weitere nicht mit dem verwendeten Antikörper eine spezifische Reaktion eingehende Antigene in der Probe vermutet werden, kann anschließend eine andere Antikörperlösung eingespeist werden. Zuvor können gegebenenfalls nachgewiesene Präzipitate durch Spülen aus der Messkammer entfernt werden.

Die nachfolgenden Beispiele dienen zur Erläuterung der vorliegenden Erfindung. Eine Einschränkung des Schutzbereichs der vorliegenden Erfindung auf den Gegenstand der Beispiele ist jedoch in keinster Weise beabsichtigt.

## Beispiele

### Beispiel 1: BSE-Bluttest

Der Rinderbestand in Deutschland beträgt etwa 15 Millionen. 2 - 3 Millionen BSE-Tests (ELISA und Western Blot) werden jährlich in Deutschland an den Gehirnen von toten Tieren durchgeführt. Ein BSE-Schnelltest (ELISA oder Western Blot) dauert zur Zeit 6 - 8 Stunden. Mit dem erfindungsgemäßen Verfahren kann der BSE-Erreger am lebenden Tier mit nur einem Tropfen Blut innerhalb von zwei Minuten nachgewiesen werden, die Kosten betragen nur einen Bruchteil.

### Beispiel 2: Blutkonserven auf neue Greutzfeld-Jakob-Krankheit (nCJK) überprüfen

Um die Bevölkerung vor dem denkbaren Risiko einer nCJK-Übertragung über das Blut zu schützen, bieten sich verschiedene Maßnahmen an. Der erfolgversprechendste Schritt wäre das Testen jeder einzelnen Blutspende auf die Infektion, wie bei Hepatitis und Aids. Doch der Erreger kommt bei Kranken nur in sehr geringen Mengen vor. Zuverlässige Tests gibt es im Stand der Technik nicht.

Das erfindungsgemäße Verfahren ist in der Lage, den Erreger im Blut bzw. in Blutprodukten nachzuweisen.

### Beispiel 3: Anwendungen in der Lebensmittelindustrie

Mykotoxine sind hochgiftige Abbauprodukte von bestimmten Pilzen. Mykotoxine sind Haptene und sind deshalb mit dem oben beschriebenen Messverfahren qualitativ und quantitativ nachweisbar.

Bei Screening-Untersuchungen von beispielsweise ganzen Schiffsladungen von Kaffee, Tee, Mehl oder Nüssen ist ein qualitativer Test, der in etwa zwei Minuten vor Ort durchgeführt werden kann, ausreichend.

Um festzustellen, ob ein Tier illegal mit Hormonen gemästet wurde, muss eine Fleischprobe auf etwa 15 verschiedene in Frage kommende Hormone untersucht werden. Auch Hormone sind Haptene und sind mit dem erfindungsgemäßen Verfahren qualitativ und quantitativ nachweisbar. Auch für beispielsweise Schwangerschaftstests und Schilddrüsen-Untersuchungen werden ebenfalls Hormontests benötigt.

Da größere Schlachtereien 2000-3000 Rinder pro Monat verarbeiten, sind etwa 50-100 Stichproben nötig, um illegales Mästen aufzudecken. Momentan liegt der Preis für einen Hormontest (auf 15 verschiedene Hormone) bei 600 Euro pro Stück Fleisch. Deshalb werden von den Fleischereien nur 5-10 Stichproben pro Monat durchgeführt, was viel zu wenig ist, um ein illegales Mästen aufzuspüren. Bei Gesprächen mit Großschlachtereien wurde ein Interesse an einer 10fach größeren Anzahl preiswerterer Tests (für ca. 60-70 Euro pro Stück Fleisch) auf Basis des erfindungsgemäßen Verfahrens signalisiert. Die herkömmlichen Hormontests erreichen diesen Preis keinesfalls.

### Beispiel 4: Nachweis von Pflanzenschutzmitteln

Tubulin-Monomere sind einzelne kleine Eiweißkügelchen, die durch eine Reaktion mit dem Energieträger GTP zu langen Ketten wachsen. Hemmstoffe und andere Toxine behindern beziehungsweise verhindern dieses Kettenwachstum. Einzelne Ketten verschlingen sich zu tauartigen Strukturen, sog. Protofilamente. Diese Protofilamente verbinden sich ihrerseits zu Mikrotubuli, die eine entscheidende Rolle bei der Zellteilung spielen. Durch die Hinderung der Monomere zur Kettenbildung beeinflusst man die Zellteilung und tötet den Schädling.

Umgekehrt bietet dieses Verfahren auch die Möglichkeit, Rückstände von Pflanzenschutzmitteln in Lebensmitteln aufzuspüren und so einen erhöhten Verbraucherschutz zu gewährleisten. Mit dem erfindungsgemäßen Verfahren kann man das oben beschriebene Kettenwachstum bzw. das Verhindern desselben direkt messen.

## Patentansprüche

1. Verfahren zum Nachweisen von Partikelmengen im femto- und attomolaren Bereich durch Erfassung von Antigen-Antikörper-Präzipitaten, welches umfasst:

Bereitstellen eines Probenfluids, das im wesentlichen Partikel mit einer bestimmten maximalen Partikelgröße enthält, wobei die Partikel mindestens zwei Antikörper-Bindestellen aufweisen; Bereitstellen eines Antikörper enthaltenden Fluids, das im wesentlichen Partikel einer bestimmten maximalen Partikelgröße enthält; Kontaktieren des Probenfluids mit dem Antikörper enthaltenden Fluid, wobei ein Reaktionsfluid erhalten wird, wobei der Antikörper in Gegenwart eines Partikels mit mindestens zwei Antikörper-Bindestellen ein Antigen-Antikörper-Präzipitat bilden kann;

Führen eines Lichtstrahls durch das Reaktionsfluid;

Erfassen eines Signals mittels einer Extinktionsmessung an der Hell-Dunkel-Grenze des Lichtkegels, der beim Durchgang des vom Laser erzeugten Lichts durch die das Reaktionsfluid enthaltende Messkammer entsteht, durch einen Photoempfänger, wobei die Signalstärke von der Größe und Zahl der gebildeten Antigen-Antikörper-Präzipitate abhängig ist, und wobei Zahl und Größe der Partikel durch die Messung der Transitionszeit bestimmt wird.

2. Verfahren nach Anspruch 1, wobei der Schritt des Bereitstellens eines Probenfluids, das im wesentlichen Partikel mit einer bestimmten maximalen Partikelgröße enthält, umfasst:

     a) Bereitstellen eines Fluids,

         Zuführen einer Probe zu dem Fluid, und Abtrennen von Partikeln, die eine bestimmte Partikelgröße überschreiten zur Gewinnung eines Probenfluids, das im wesentlichen lediglich Partikel mit einer bestimmten maximalen Partikelgröße enthält, oder

     b) Bereitstellen eines Fluids, das im wesentlichen Partikel mit einer bestimmten maximalen Partikelgröße enthält, und

         Zuführen einer Probe zu dem Fluid, die im wesentlichen Partikel mit einer bestimmten maximalen Partikelgröße enthält, zur Gewinnung eines Probenfluids, das im wesentlichen Partikel mit einer bestimmten maximalen Partikelgröße enthält.

3. Verfahren nach einem der vorstehenden Ansprüche 1 oder 2, wobei die Abtrennung der Partikel mit einer Größe oberhalb der bestimmten maximalen Partikelgröße durch Filtration erfolgt, wobei der Filter vorzugsweise eine Porengröße von 20 - 450 nm, bevorzugter von 100 - 300 nm, insbesondere von 200 nm aufweist.

4. Verfahren nach einem der vorstehenden Ansprüche 1 bis 3, wobei als Antikörper mindestens zwei monoklonale Antikörper oder ein polyklonaler Antikörper eingesetzt werden.

5. Verfahren nach einem der vorstehenden Ansprüche 1 bis 4, wobei der Antikörper ausgewählt ist aus der Gruppe bestehend aus Immunglobulin G oder Immunglobulin M.

6. Verfahren nach einem der vorstehenden Ansprüche 1 bis 5, wobei das Verfahren eine quantitative oder halb-quantitative Erfassung der Partikelmenge ermöglicht.

7. Verfahren nach einem der vorstehenden Ansprüche 1 bis 6, wobei bei konstanter Antikörperkonzentration die Abnahme des Meßsignals im direkten Zusammenhang mit der Antigenkonzentration steht.

8. Computerprogramm-Produkt, welches Programmcode-Mittel umfasst, die auf einem Computer-lesbaren Medium gespeichert sind, zur Ausführung des Verfahrens nach einem der Ansprüche 1 bis 7, wenn das Computerprogramm-Produkt auf einem Computer, einer Netzwerk-Vorrichtung oder einer Vorrichtung, insbesondere einer analytischen Erfassungsvorrichtung läuft.

9. Computerprogramm-Produkt, welches einen Programmcode umfasst und von einem Server herunterladbar ist, zur Ausführung des Verfahrens nach einem der Ansprüche 1 bis 7, wenn das Computerprogramm-Produkt auf einem Computer, einer Netzwerk-Vorrichtung oder einer Vorrichtung, insbesondere einer analytischen Erfassungsvorrichtung läuft.

10. Kit zum qualitativen und/oder quantitativen Nachweis eines bestimmten, nachzuweisenden Partikels, wobei das bestimmte Partikel mindestens zwei Antikörper-Bindestellen aufweist, wobei der Kit umfasst:

     mindestens einen Antikörper, der spezifisch an das bestimmte Partikel binden kann, und mindestens ein geeignetes Fluid zur Aufnahme der Probe, und eine Vorrichtung zur Erfassung geringer Partikelmengen, welche aufweist:

         einen Laser, eine Messkammer, und einen Photoempfänger, der zur Durchführung einer Extinktionsmessung an der Hell-Dunkel-Grenze des Lichtkegels ausgelegt ist, der bei Durchgang des vom Laser erzeugten Lichts durch die Partikel in einem Fluid enthaltende Messkammer entsteht, wobei der Photoempfänger bezüglich des Lasers derart angeordnet ist, dass der Laserstrahl knapp am Photoempfänger vorbei geht.

## Claims

1. Method of determining particle quantities in the femto- and attomolar range by detecting antigen-antibody precipitates, which comprises:

providing a sample fluid, which contains substantially particles of a specific maximum particle size, wherein the particles have at least two antibody-binding points;
providing an antibody-containing fluid, which contains substantially particles of a specific maximum particle size;
contacting the sample fluid with the antibody-containing fluid, wherein a reaction fluid is obtained, wherein the antibody in the presence of a particle having at least two antibody-binding points may form an antigen-antibody precipitate;
guiding a light beam through the reaction fluid;
acquiring a signal by means of an extinction measurement at the light/dark boundary of the light cone, which arises when the light generated by the laser passes through the measuring chamber containing the reaction fluid, by means of a photoreceiver, wherein the signal intensity is dependent upon the size and number of the formed antigen-antibody precipitates, and wherein the number and size of the particles is determined by measurement of the transition time.

2. Method according to claim 1, wherein the step of providing a sample fluid, which contains substantially particles of a specific maximum particle size, comprises:

a) providing a fluid,

supplying a sample to the fluid, and separating particles that exceed a specific particle size in order to obtain a sample fluid, which contains substantially only particles of a specific maximum particle size, or

b) providing a fluid, which contains substantially particles of a specific maximum particle size, and

supplying a sample to the fluid, which contains substantially particles of a specific maximum particle size, in order to obtain a sample fluid, which contains substantially particles of a specific maximum particle size.

3. Method according to one of the preceding claims 1 or 2, wherein the separation of the particles of a size above the specific maximum particle size is effected by filtration, wherein the filter preferably has a pore size of 20 - 450 nm, more preferably of 100 - 300 nm, in particular of 200 nm.

4. Method according to one of the preceding claims 1 to 3, wherein as an antibody at least two monoclonal antibodies or one polyclonal antibody is used.

5. Method according to one of the preceding claims 1 to 4, wherein the antibody is selected from the group comprising immunoglobulin G or immunoglobulin M.

6. Method according to one of the preceding claims 1 to 5, wherein the method enables a quantitative or semi-quantitative detection of the particle quantity.

7. Method according to one of the preceding claims 1 to 6, wherein, given a constant antibody concentration, the decrease of the measuring signal is in direct relationship to the antigen concentration.

8. Computer program product, which comprises program code means that are stored on a computer-readable medium, for effecting the method according to one of claims 1 to 7 when the computer program product runs on a computer, a network device or a device, in particular an analytical detection device.

9. Computer program product, which comprises a program code and is downloadable from a server, for effecting the method according to one of claims 1 to 7 when the computer program product runs on a computer, a network device or a device, in particular an analytical detection device.

10. Kit for the qualitative and/or quantitative determination of a specific particle to be determined, wherein the specific particle has at least two antibody-binding points, wherein the kit comprises:

at least one antibody, which may bind specifically to the specific particle, and
at least one suitable fluid for receiving the sample, and
a device for detecting low particle quantities, which comprises:
a laser,
a measuring chamber, and
a photoreceiver, which is designed to carry out an extinction measurement at the light/dark boundary of the light cone that arises when the light generated by the laser passes through the measuring chamber containing particles in a fluid, wherein the photoreceiver is disposed in such a way relative to the laser that the laser beam barely passes the photoreceiver.

**Revendications**

1. Procédé pour la détection de quantités de particules de l'ordre de la femtomole et de l'attomole par la mesure de précipités antigène - anticorps, qui consiste à :

préparer un fluide échantillon, qui contient sensiblement des particules ayant une granulométrie maximale déterminée, les particules présentant au moins deux sites de liaison aux anticorps ;

préparer un fluide contenant des anticorps, qui contient sensiblement des particules d'une granulométrie maximale déterminée ;

mettre en contact le fluide échantillon avec le fluide contenant les anticorps, un fluide réactionnel étant obtenu, dans lequel l'anticorps peut former en présence d'une particule ayant au moins deux sites de liaison aux anticorps un précipité antigène - anticorps ;

conduire un faisceau lumineux à travers le fluide réactionnel ;

détecter un signal au moyen d'une mesure d'extinction à la limite clair - obscur du cône lumineux, qui apparaît lors du passage de la lumière produite par le laser à travers la chambre de mesure contenant le fluide réactionnel, par le biais d'un photorécepteur, la force du signal dépendant de la taille et du nombre de précipités antigène - anticorps formés, et le nombre et la taille des particules étant déterminés par la mesure du temps de transition.

2. Procédé selon la revendication 1, dans lequel l'étape de préparation d'un fluide échantillon, qui contient sensiblement des particules ayant une granulométrie maximale déterminée, consiste à :

a) préparer un fluide,

amener un échantillon au fluide, et séparer les particules qui dépassent une granulométrie déterminée pour obtenir un fluide échantillon qui contient sensiblement uniquement des particules ayant une granulométrie maximale déterminée, ou

b) préparer un fluide, qui contient sensiblement des particules ayant une granulométrie maximale déterminée, et

amener un échantillon au fluide, qui contient sensiblement des particules ayant une granulométrie maximale déterminée, pour obtenir un fluide échantillon qui contient sensiblement des particules ayant une granulométrie maximale déterminée.

3. Procédé selon l'une des revendications précédentes 1 ou 2, dans lequel la séparation des particules ayant une taille située au-dessus de la granulométrie maximale déterminée s'effectue par filtration, où le filtre présente de préférence une taille de pores de 20 à 450 nm, plus préférentiellement de 100 à 300 nm, en particulier de 200 nm.

4. Procédé selon l'une des revendications précédentes 1 à 3, dans lequel on met en oeuvre à titre d'anticorps au moins deux anticorps monoclonaux ou un anticorps polyclonal.

5. Procédé selon l'une des revendications précédentes 1 à 4, dans lequel l'anticorps est choisi dans le groupe constitué de l'immunoglobuline G ou de l'immunoglobuline M.

6. Procédé selon l'une des revendications précédentes 1 à 5, dans lequel le procédé permet une détection quantitative ou semi-quantitative de la quantité de particules.

7. Procédé selon l'une des revendications précédentes 1 à 6, dans lequel, pour une concentration en anticorps constante, la diminution du signal de mesure est en relation directe avec la concentration en antigène.

8. Logiciel informatique, qui comprend des moyens de codage logiciel, qui sont enregistrés sur un support lisible par informatique, pour l'exécution du procédé selon l'une des revendications 1 à 7, quand le logiciel informatique fonctionne sur un ordinateur, un dispositif en réseau ou un dispositif, en particulier un dispositif de détection analytique.

9. Logiciel informatique, qui comprend un code logiciel et peut être téléchargé sur un serveur, pour l'exécution du procédé selon l'une des revendications 1 à 7, quand le logiciel informatique fonctionne sur un ordinateur, un dispositif en réseau ou un dispositif, en particulier un dispositif de détection analytique.

10. Kit pour la détection qualitative et/ou quantitative d'une particule déterminée à détecter, où la particule déterminée comprend au moins deux sites de liaison aux anticorps, le kit comprenant :

au moins un anticorps, qui peut lier spécifiquement la particule déterminée, et au moins un fluide approprié pour accueillir l'échantillon, et un dispositif pour la détection de faibles quantités de particules, qui comprend :

un laser, une chambre de mesure, et

un photorécepteur, qui est conçu pour réaliser une mesure d'extinction à la limite clair - obscur du cône lumineux, qui apparaît lors du passage de la lumière produite par le laser à travers la chambre de mesure contenant les particules dans un fluide, le photorécepteur étant disposé par rapport au laser de manière à ce que le faisceau laser passe juste devant le photorécepteur.

FIG. 1a - d

Fig. 2a

Fig. 2b

**FIG. 3**